# EUROPEAN PATENT APPLICATION

(11) **EP 3 940 079 A1**
(43) Date of publication of application: **19.01.2022**
(21) Application number: 20185981.6
(22) Date of filing: 15.07.2020
(51) Int. Cl.: C12N 15/90, C12N 15/65, C12N 15/113

(54) **A STREAMLINED SELECTION METHOD FOR THE GENOME EDITING OF CELLS**

(71) Applicant: IUF Leibnitz-Institut für Umweltmedizinische Forschung gGmbH, 40225 Düsseldorf (DE)
(72) Inventor: ROSSI, Andrea, 40225 Düsseldorf (DE)
(74) Representative: dompatent von Kreisler Selting Werner - Partnerschaft von Patent- und Rechtsanwälten mbB

(57) **Abstract**

The present application refers to a method for enriching genetically modified cells comprising the following steps:
a) providing a vector comprising a sequence of an enzyme capable of cutting DNA and a sequence of a surface marker;
b) transfecting cells with the vector resulting in genetically modified cells which at the same time express the enzyme capable of cutting DNA, and the surface marker;
c) enriching the genetically modified cells by magnetic sorting using the surfaced marker.

## Description

The present invention relates to the enrichment of genetically modified cells through a surface marker and magnetic sorting. Magnetic beads can selectively bind to this surface marker.

Genome editing technologies have, in the last decade, substantially improved the ability to make precise changes in the genomes of eukaryotic cells. Programmable nucleases, such as meganucleases derived from microbial mobile genetic elements as Zinc Finger (ZF) and Transcription activator-like effector nucleases (TALENs) have been used with discrete success to modify the genome of different species (Gupta and Musunuru 2014). Few years ago, CRISPR/Cas system revolutionized the ability to interrogate the function of the genome. CRISPR/Cas was shown to be potentially useful clinically to correct genetic DNA mutations to treat diseases that are refractory to traditional therapies or where therapies are not yet available (Cong et al., 2013; Mali et al., 2013a.)

The main limiting factor for many labs, when using the CRISPR/Cas system but also other systems, is the ability to sort transfected cells that carry the desired mutation.

The enrichment of genetically modified cells after transfection is mainly achieved by antibiotic or fluorescent sorting. Both selection strategies are routinely used in most cells and molecular biology laboratories because they are relatively easy to perform and the results are reproducible. However, there are several limitations that must be taken into consideration when choosing the selection method.

Antibiotics are affordable, and can be used without specific equipment, but the cell selection requires time, it is laborious and low-throughput when compared with other selection methods. The first critical step, when using antibiotics, is to determine the optimal concentration, titration, to use for the selection of stable colonies. The sub-lethal concentration can be determined with the calibration of a killing curve in order to kill not transfected cells without affecting the transfected ones. Furthermore, antibiotics need few days to kill cells that are not carrying the antibiotic resistant gene, making the process of generating genetically modified cells longer. Last but not least one of the main issues connected to the use of antibiotics is the emergence of spontaneous resistant clones that do not curry the gene of interest (Choudhury 2017).

Antibiotics-free selection using FACS can be an alternative to avoid the above issues. FACS has significantly increased the volume of cells that can be screened in a short period of time (Browne and AI-Rubeai 2007). Unfortunately, florescent markers require large, complex, expensive instrumentation typically operated by highly trained, often dedicated, specialist. The ability to quickly and simultaneously query multiple parameters on large numbers of individual cells is generally reserved for shared core facilities or well-equipped specialized laboratories.

One of the big asset of FACS is that it allows a good cell selection based on multiparameter separations and, if this is a big advantage on one side, on the other side it can lead to inaccuracy when it comes to discriminate between cell populations with closely related immunophenotypes due to the fact that the spectra of fluorophores can overlap (Choudhury 2017). Sometimes this technology is limited by the absence of available specific antibodies or by the need to optimise experimental conditions to a specific cell line (Browne and Al-Rubeai 2007).

There is therefore a need to select the effectively transfected cells and thus to enrich the genetically modified cells after transfection which avoids the deficiencies of the prior art. The method should be fast and affordable without the requirement of a dedicated facility. Further, the viability of recovered cells should be good. Additionally, the selection method should have a high specificity to the transfected cells.

Surprisingly it was found that magnetic sorting enables high selectivity without the need of dedicated facilities but at the same time high viability of recovered cells. The object of the present invention is therefore solved by a method for enriching genetically modified cells comprising the following steps:
a) providing a vector comprising a sequence of an enzyme capable of cutting DNA and a sequence of a surface marker;
b) transfecting cells with the vector resulting in genetically modified cells which at the same time express the enzyme capable of cutting DNA, and the surface marker,
c) enriching the genetically modified cells by magnetic sorting using the surfaced marker.

Surprisingly it was found that it is possible in one step to get a double positive transfected cell, which at the same time comprises the gene editing as well as the newly introduced surface marker. By magnetic sorting it is possible to get a high throughput selection method with high selectivity to isolate the target populations. The method is easy to perform and there is a high viability of the recovered cells. The method according to the present invention is further cheap, easy to perform and result in reproducible results. Further, the method is highly versatile and can thus be extended to a wide range of cells.

The vector is provided such that the enzyme and the surface marker are still in an active form after transfecting a cell with it. Thus, the enzyme capable of cutting DNA is active within the cell to perform the genetic engineering. The enzyme cuts DNA at a predetermined position. At this position, the DNA provided additionally in step a) is introduced into the DNA of the cell at the position of the cut, resulting in a genetically modified cell.

In the method of the present invention, the cell will not only be modified via the enzyme and DNA but will also be able to express a surface marker, which was also introduced via the vector. Accordingly, with the method according to the present invention it is possible to obtain a cell being double positive for the enzyme and the surface marker by introducing only one vector.

The enzyme capable of cutting DNA is preferably an endonuclease. Especially preferred this endonuclease is selected from Fok1, Cas9, Cas12a, Cas12b and Cas13.

For the first time it is possible to sort genetically modified cells without leaving the cell culture room. Step a) of the method according to the present invention accordingly encompasses providing an enzyme capable of cutting DNA as well as providing a surface marker. Both, enzyme and surface marker are introduced vie the vector into the cell resulting in a double positive cell. The enzyme is amended by fusing the surface marker to one end of the enzyme, such that after transfection of the cell with the fused enzyme -surface marker both of the enzyme and the surface marker are active inside the cell. This vector, comprising at least both of the enzyme and the surface marker is introduced into the cell. This method is a one-step genome editing which at the same time enables successful sorting of the cells. It allows the control of successful sorting immediately without further manipulation of the cell.

The enzyme capable of cutting DNA is preferably an endonuclease. This allows genome editing for example via zinc-finger nuclease (ZFNs), transcription activator-like effector nuclease (TALENs) or clustered regularly interspaced short palindromic repeats (CRISPR). The enzyme preferably selected to perform the genome editing is accordingly selected from Fok1, Cas9, Cas12a, Cas12b and Cas13. In one preferred embodiment, it is selected from Cas9, Cas12 and Cas13; especially preferred it is selected from Cas12a, Cas12b and Cas13, preferably it is Cas9. The CRISPR/Cas9 genome editing is preferred as it enables an easy genome editing with different kinds of gRNAs to a wide range of cells.

Thus, in a preferred embodiment, the method of the present invention comprises the following steps:
a) providing a vector comprising Cas9 and a surface marker fused to one terminus of Cas9, preferably the c-terminus;
b) transfecting cells with this vector resulting in genetically modified cells which at the same time express the surface marker,
c) enriching the genetically modified cells by magnetic sorting using the surfaced marker.

This preferred method according to the present invention thus enables to obtain double positive Cas9/surface marker cells in one step.

The surface marker in any embodiment is preferably one which does not occur naturally in the transfected cells, so that the sorting has a high specificity to the newly introduced surface marker. Especially, the cells to be transfected are eukaryotic cells and the surface marker is a marker not naturally occurring in eukaryotic cells. Alternatively, the cells to be transfected are induced pluripotent stem cells and the surface marker is a marker not naturally occurring in induced pluripotent stem cells.

Cells which may be treated with a method according to the present invention are for example induced pluripotent stem cells, primary human fibroblasts, HaCaT, and HEK cells. The surface marker can be any surface marker known by the skilled artisan. It may be selected from CD 19, tCD19, CD4, and CD 3. But also unrelated surface markers that have an external epitope that can be targeted with an antibody. CD19 and tCD19 are preferred as they occur naturally only at B-cells, so that this surface marker can be used for a broad range of different cell types.

The method of the invention thus enables to obtain genome edited cells with a high selectivity in a fast and easy manner. It is possible to transfect cells for example with gRNA targeting for human genes, *CPAP* (centromere protein J), *UGT1A* (UDP glucuronosyltransferase family 1 member A1), *DMD* (dystrophin), *ERGCC6* (ERCC excision repair 6, a chromatin remodeling factor) and *ACTB* (beta actin). These molecules are introduced together with the vector into the cell in step a) of the method according to the present invention. Accordingly, in a preferred embodiment, the method of the present invention comprises the following steps:
a) providing a vector comprising a sequence of an enzyme capable of cutting DNA and a sequence of a surface marker, and providing a gRNA to genetically modify a cell;
b) transfecting cells with the vector and the gRNA resulting in genetically modified cells which at the same time express the enzyme capable of cutting DNA, the surface marker as well as the gRNA;
c) enriching the genetically modified cells by magnetic sorting using the surfaced marker.

The gRNA can for example be selected from gRNA targeting for human genes, *CPAP* (centromere protein J), *UGT1A* (UDP glucuronosyltransferase family 1 member A1), *DMD* (dystrophin), *ERGCC6* (ERCC excision repair 6, a chromatin remodeling factor) and *ACTB* (beta actin).

In a further preferred embodiment, the method of the present invention comprises the following steps:
a) providing a vector comprising Cas9 and a surface marker fused to one terminus of Cas9, preferably the c-terminus; and providing a gRNA to genetically modify a cell;
b) transfecting cells with this vector and the gRNA resulting in genetically modified cells which at the same time express the enzyme capable of cutting DNA, the surface marker as well as the gRNA;
c) enriching the genetically modified cells by magnetic sorting using the surfaced marker.

The method of the present invention further comprises the step of adding magnetic beads to the transfected cells. The beads are crosslinked with a suitable antibody such that they selectively bind to the surface marker. The beads may for example be modified with an antibody directed against the surface marker. In one preferred embodiment, the magnetic beads are therefore modified with an antibody against CD19 or an antibody against tCD19.

After the beads have successfully attached to the surface marker of the cells, the sorting in step e) is performed in a high-gradient magnetic field obtained by a permanent magnet. To do so, the cells are in suspension. This suspension is added to a column and a buffer is used to elute the cells from the column. The buffer used for elution preferably consists of Dulbecco's Phosphate-buffered saline, EDTA and fetal bovine serum, wherein said buffer is not necessarily degassed prior to elution. Instead of fetal bovine serum (FBS), also BSA (bovine serum albumin) may be used, but FBS is preferred.
In the following the method of the present invention is further explained in a non-limiting manner by examples, in which different types of cells are transfected and sorted with the method according to the present invention.

### Examples:

If not indicated otherwise, all transfection steps were carried out by using Cas9 as restriction enzyme. The surface marker used was tCD19 which was fused at the c-terminus of the Cas9 plasmid, spaced by the self-cleaving peptide 2A.

### Example 1:

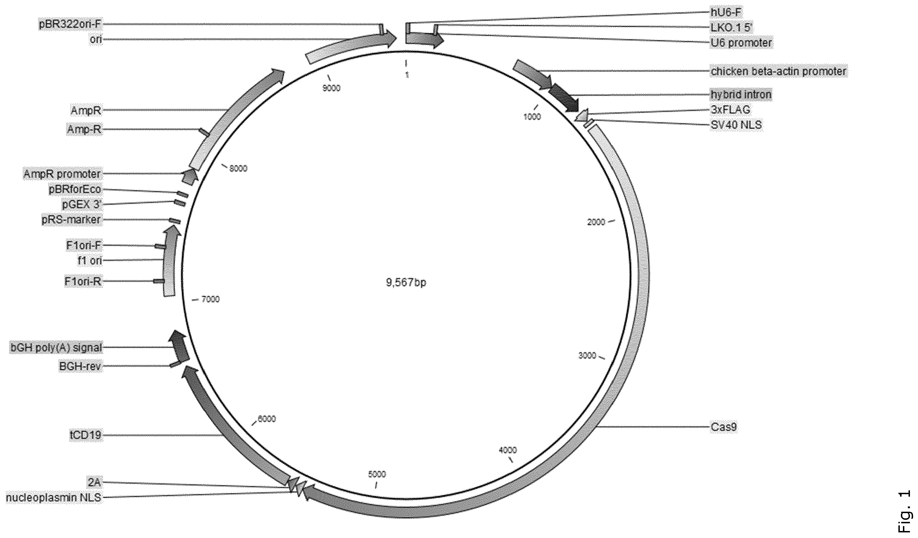

A truncated CD19 was fused at the c-terminus of a spCas9-2A plasmid (see Fig. 1). HEK cells were transfected with a Cas9-2A-GFP (as reference) and Cas9-2A-CD19 (according to the invention). Successfully transfected cells will express a functional Cas9, able to go into the nucleus to mediate genome editing in the presence of a gRNA, and a surface marker, tCD19 to magnetically sorted transfected cells (According to the invention) or GFP for fluorescence sorting (reference example).

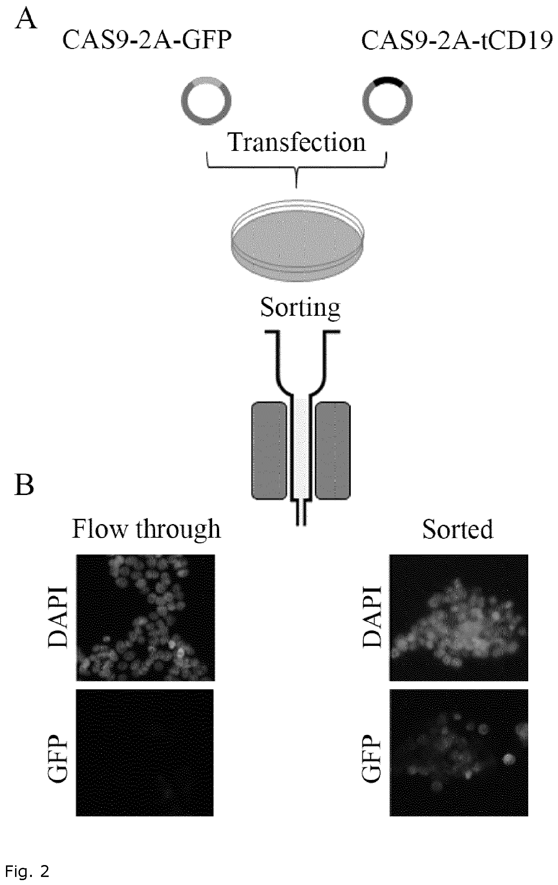

Fig. 2A shows schematically the comparison between a GFP fluorescence according to the prior art and a magnetic sorting according to the present invention.

After magnetic sorting, the majority of CD19 positive cells were GFP positive whereas flow through cells, no or little GFP fluorescence was detected (Fig. 2B).

### Example 2:

Different gRNAs were designed and cloned targeting four human genes, CPAP, *UGT1A1, DMD* and *ACTB.* These has the following sequences:

gene | gRNA | PAM
UGTA1A ex3 | GTTCGCAAGATTCGATGGTC | GGG
DMD ex55 | ATGTCCTACAGGATGCTACC | TGG
ACTB ex4 | TGGATAGCAACGTACATGGC | TGG
CPAP ex17 | GGGTAATGTGCTAATGGACA | CGG

HEK cells were transfected with these gRNAs using Cas9 modified with tCD19. The cells were sorted after 48 hrs.

In order to assess the genome editing efficiency, High-Resolution Melting (HRM) curve analysis was used. For reference, also wild-type (WT) cells were analysed.

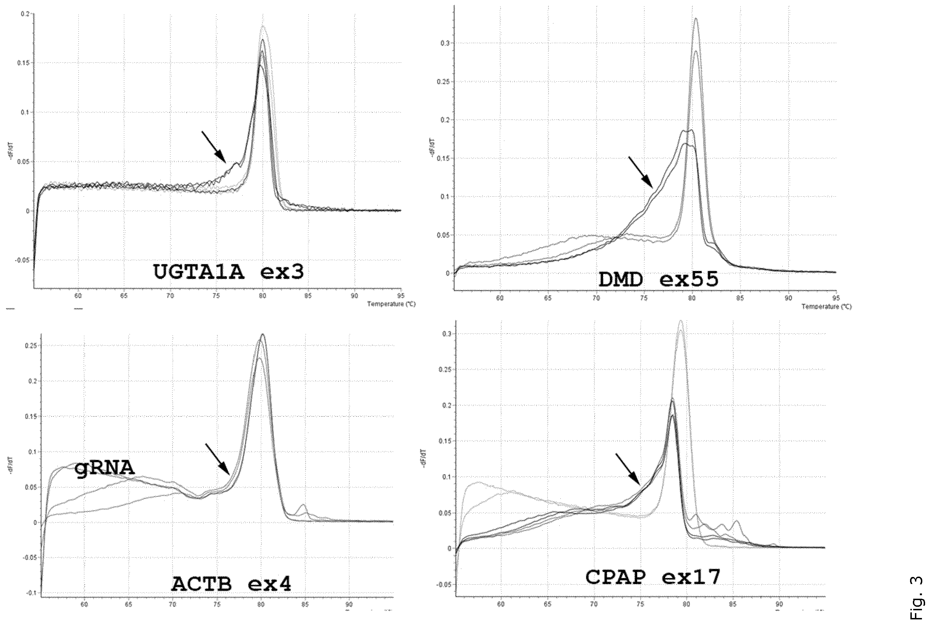

WT cells as well as CD19 positive cells were analysed by HRMA. RMA. A clear single peak was detected for WT cells indicating the lack of the transfected indels. In contrast CD19 positive cells displayed after sorting the typical irregular curve synonym of DNA base indels (Fig. 3).

### Example 3:

The Cas9 CD19 double positive transfected cells of Example 2 were additionally analysed after sorting with next generation sequencing and Sanger sequencing.

The measurements revealed the successful generation of indels indicating the efficacy of each gRNA and MAC system to sort genome edited cells:

UGT1A1 indels

wt CTGGAACCCGACCATCGAATCTTGCGAACAACACGATACT
Δ8 CTGGAACCC--------AATCTTGCGAACAACACGATACT
Δ15 CTGGAACC---------------GCGAACAACACGATACT

wt ACAACTGCCAATGTCCTACAGGATGCTACCCGTAAGGAAA
Δ9 ACAACTGCCAATG---------ATGCTACCCGTAAGGAAA
Δ19 ACAA-------------------TGCTACCCGTAAGGAAA

ACTB indels

wt TTCAACACCCCAGCCATGTACGTTGCTATCCAGGCTGTGC
Δ6 TTCAACACCCCAGCCATG------GCTATCCAGGCTGTGC
Δ7 TTCAACACCCCAGCCATG-------CTATCCAGGCTGTGC

CPAP indels

wt AGGACAAGGAGGGTAATGTGCTAATGGACACGGAGCTGTG
Δ2 AGGACAAGGAGGGTAATGTGCTAATGGA--CGGAGCTGTG
Δ10 AGGACAAGGAGGGTAAT------------ACGGAGCTGTG

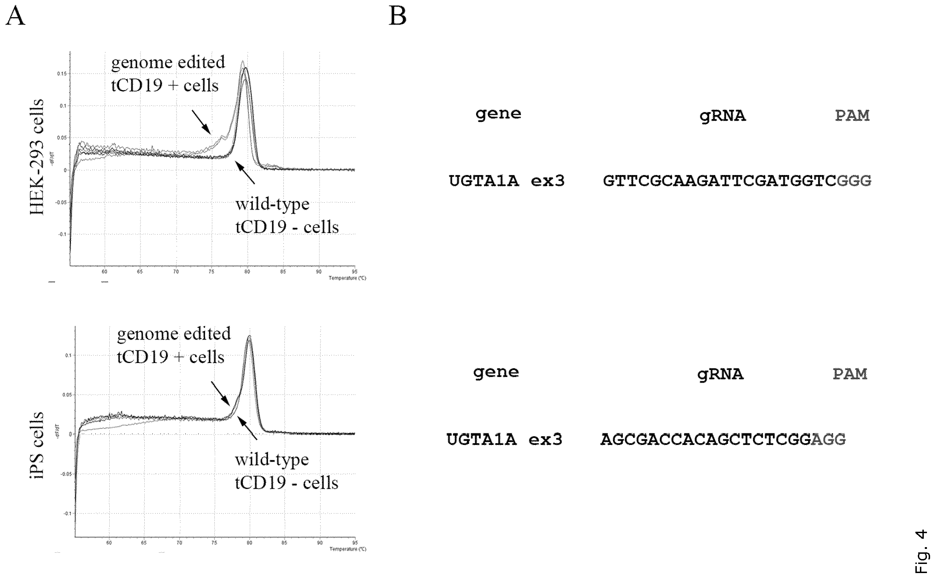

Moreover, while mutations were detected in magnetic sorted cells, mutations were not detected in the flow through, as can be seen from Fig. 4.

### Example 4:

Human primary fibroblasts were transfected with a gRNA targeting DMD:

gene | gRNA | PAM
DMD ex55 | ATGTCCTACAGGATGCTACC | TGG

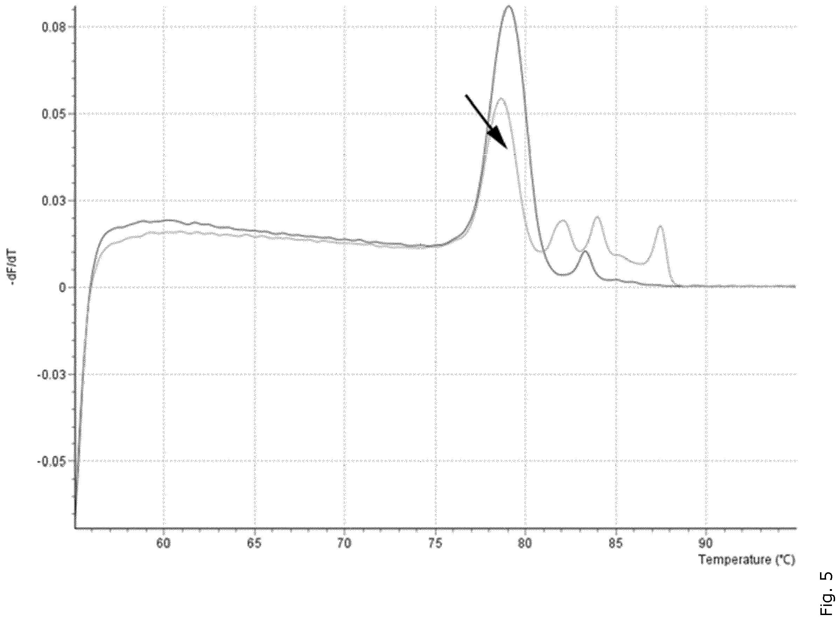

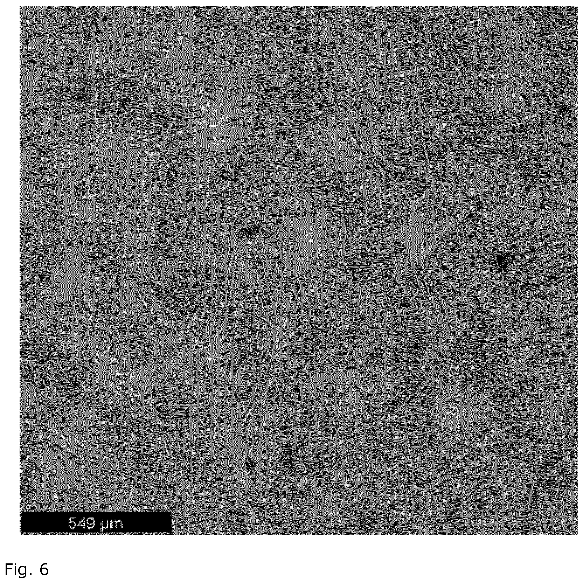

After transfection, cells were magnetic sorted after two days, part was plated on a new dish and part used to extract genomic DNA for HRM analysis (Fig. 5 shows the results of the HRM analysis). Indels were detected in CD19 positive cells by HRMA and SANGER sequencing (Fig. 5). Interestingly, magnetic sorting according to the invention did not affect the health of human primary fibroblasts, as can be seen from Fig. 6, being a picture of the transfected and sorted cells with an optical microscope.

### Example 5:

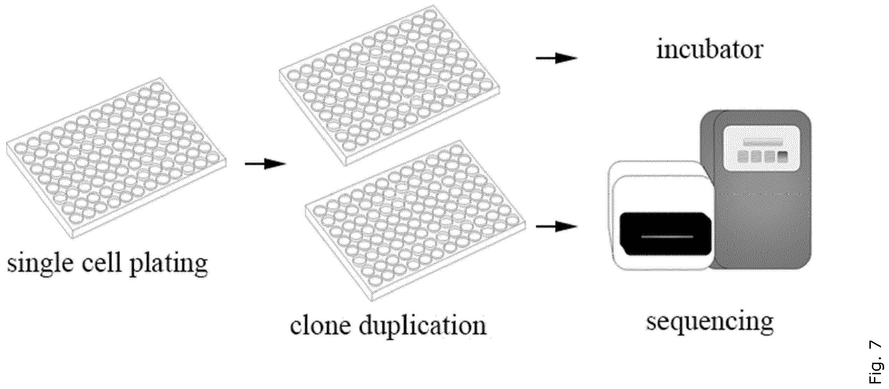

Two gRNA targeting *UGTA1* and *ERCC6* were used to transfect iPSCs. After 48 hours they were magnetically sorted. Single cells, in presence of ROCK inhibitors, were plated in a 96 multi-well and duplicated after a week. One plate was digested with Prot-K for further genotyping analysis and the other one was kept in the incubator, as schematically depicted in Fig. 7.

Deep sequencing analysis was performed as previously described using an Illumina miSeq sequencer (JL Schmid-Burgk et al., 2014). The data were analyzed using online available software
(http://www.outknocker.org/outknocker2.htm) and revealed the presence and type of mutation for each clone:

UGT1A1 indels

REFERENCE GTAGCCACTTAACAAGTATCGTGTTGTTCGCAAGATTCGATGGTCGGGTTCCAGTGTACCGCCACAGGACCTGGGAGGGG

CALL #1 GTAGCCACTTAACAAGTATCGTGTTGTTCGCAAGATTCG.......GGTTCCAGTGTACCGCCACAGGACCTGGGAGGGG 100%
7nt deletion 162 reads

REFERENCE GGGTAGCCACTTAACAAGTATCGTGTTGTTCGCAAGATTCGATGGTCGGGTTCCAGTGTACCGCCACAGGACCTGGGAGG

CALL #1 GGGTAGCCACTTAACaAGTATCGTGTTGTTCGCAAGATTCGATG.........CAGTGTACCGCCACAGGACCTGGGAGG 99%
9nt deletion 757 reads

REFERENCE TGGGGTAGCCACTTAACAAGTATCGTGTTGTTCGCAAGATTCGATGGTCGGGTTCCAGTGTACCGCCACAGGACCTGGGA

CALL #1 TGGGGTAGCCACTTAACAAGTATCGTGTTGTTCGCAAGATTCGATGGTCGGGTTCCAGTGTACCGCCACAGGACCTGGGA 51%
1nt insertion T 753 reads

CALL #2 TGGGGTAGCCACTTAACAAGTATCGTGTTGTTCGCAAGATTCGATGGTCGGGTTCCAGTGTACCGCCACAGGACCTGGGA 47%
1nt insertion G 684 reads

ERCC6 indels

REFERENCE CCTAACTTTCCCGGGAAGATGAAGTCAAAGAGCGACCACAGCTCTCGGAGGTTATTTTGCATCGGTGAGCCAGACAGAAT

CALL #1 CCTAACTTTCCCGGGAAGATGAAGTCAAAGAGCGACCACAGCTCTTCGGAGGTTATTTTGCATCGGTGAGCCAGACAGAAT 97%
1nt insertion T 271 reads

REFERENCE CCTAACTTTCCCGGGAAGATGAAGTCAAAGAGCGACCACAGCTCTCGGAGGTTATTTTGCATCGGTGAGCCAGACAGAAT

CALL #1 CCTAACTTTCCCGGGAAGATGAAGTCAAAGAGCGACCACAGCTCT--------ATTTTGCATCGGTGAGCCAGACAGAAT 49%
8nt deletion 154 reads

CALL #2 CCTAACTTTCCCGGGAAGATGAAGTCAAAGAGCGACCACAGCTCTTCGGAGGTTATTTTGCATCGGTGAGCCAGACAGAAT 46%
1nt insertion T 144 reads

REFERENCE CCTAACTTTCCCGGGAAGATGAAGTCAAAGAGCGACCACAGCTCTCGGAGGTTATTTTGCATCGGTGAGCCAGACAGAAT

CALL #1 CCTAACTTTCCCGGGAAGATGAAGTCAAAGAGCGACCACAGCTCTTCGGAGGTTATTTTGCATCGGTGAGCCAGACAGAAT 61%
1nt insertion T 38 reads

CALL #2 CCTAACTTTCCCGGGAAGATGAAGTCAAAGAGCGAC---------------TTATTTTGCATCGGTGAGCCAGACAGAAT 34%
15nt deletion 21 reads

The method of the present invention enables a streamlined selection of genome edited cells that is envisioned to be an incredibly useful tool. Using this method enables the skilled artisan to enrich genome edited cells of different genes in different cell types like HEK293, human primary fibroblasts and iPS cells.

Magnetic sorting of genome edited cells offers several advantages over antibiotics and FACS, both known from the prior art: 1) Faster than antibiotics, 2) Cheaper than FACS and 3) More gentle than FACS especially when dealing with sensitive cells like iPSCs. Magnetic sorting outperformed FACS in number of overall living recovered cells in all our experiments when using commercially available iPSCs IMR-90.

## Claims

1. Method for enriching genetically modified cells comprising the following steps:
a) providing a vector comprising a sequence of an enzyme capable of cutting DNA and a sequence of a surface marker;
b) transfecting cells with the vector resulting in genetically modified cells which at the same time express the enzyme capable of cutting DNA, and the surface marker;
c) enriching the genetically modified cells by magnetic sorting using the surfaced marker.

2. Method according to claim 1, wherein the enzyme is an endonuclease.

3. Method according to claim 2, wherein the endonuclease is selected from Fok1, Cas9, Cas12a, Cas12b and Cas13.

4. Method according to any of claims 1 to 3, wherein the cells are selected from induced pluripotent stem cells, primary human fibroblasts, HaCaT, and HEK cells.

5. Method according to any of claims 1 to 4, wherein the cells to be transfected are eucariotic cells and the surface marker is a marker not naturally occurring in eucariotic cells.

6. Method according to any of claims 1 to 4, wherein the cells to be transfected are induced pluripotent stem cells and the surface marker is a marker not naturally occurring in induced pluripotent stem cells

7. Method according to any of claims 1 to 6, wherein the surface marker is selected from CD 19, tCD19, CD4, CD 3.

8. Method according to any of claims 1 to 7 comprising the following steps:
a) providing a vector comprising a sequence of an enzyme capable of cutting DNA and a sequence of a surface marker, and providing a gRNA to genetically modify a cell;
b) transfecting cells with the vector and the gRNA resulting in genetically modified cells which at the same time express the enzyme capable of cutting DNA, the surface marker as well as the gRNA,
c) enriching the genetically modified cells by magnetic sorting using the surfaced marker.

9. Method according to claim 7, wherein the gRNA is selected from gRNA targeting for human genes, *CPAP* (centromere protein J), *UGT1A* (UDP glucuronosyltransferase family 1 member A1), *DMD* (dystrophin), *ERGCC6* (ERCC excision repair 6, a chromatin remodeling factor) and *ACTB* (beta actin).

10. Method according to any of claims 1 to 9, wherein the sorting in step c) is performed in a high-gradient magnetic field obtained by a permanent magnet.

11. Method according to any of claims 1 to 10, wherein the cells are sorted in a column and eluted with a buffer comprising Dulbecco's Phosphate-buffered saline, EDTA and fetal bovine serum, wherein said buffer is not degassed.
